# EUROPÄISCHE PATENTANMELDUNG

(11) **EP 4 349 386 A1**
(43) Veröffentlichungstag der Anmeldung: **10.04.2024**
(21) Anmeldenummer: 22200221.4
(22) Anmeldetag: 07.10.2022
(51) Int. Cl.: A61M 11/04, A61M 15/06, A61M 11/02, A61M 16/00

(54) **VORRICHTUNG FÜR DIE ANREICHERUNG VON LUFT MIT SAUERSTOFF**

(71) Anmelder: Wolter, Jens, 10409 Berlin (DE)
(72) Erfinder: Wolter, Jens, 10409 Berlin (DE); Pappitch, Anton, 13357 Berlin (DE)
(74) Vertreter: Hertin und Partner Rechts- und Patentanwälte PartG mbB

(57) **Zusammenfassung**

Die Erfindung betrifft eine Vorrichtung für die Anreicherung von Luft mit Sauerstoff umfassend eine Steuereinheit und mindestens einen Sensor. Die Vorrichtung umfasst eine erste Kammer und eine zweite Kammer, wobei die erste Kammer dafür konfiguriert ist, Sauerstoff unter Druck zu halten und als Reaktion auf ein erstes Signal der Steuereinheit den Sauerstoff mit einem ersten Volumenstrom abzugeben, die zweite Kammer dafür konfiguriert ist, als Reaktion auf ein zweites Signal der Steuereinheit einen inhalierbaren Dampf oder Aerosol mit einem zweiten Volumenstrom abzugeben, und der erste Volumenstrom größer als der zweite Volumenstrom ist. Die Erfindung betrifft weiterhin eine Verwendung der Vorrichtung für die Verbesserung der Luftqualität in einem Raum. In einem weiteren Aspekt betrifft die Vorrichtung einen Bausatz für die Anreicherung von Luft mit Sauerstoff.

## Beschreibung

Die Erfindung betrifft eine Vorrichtung für die Anreicherung von Luft mit Sauerstoff und für die Lieferung von Duft und/oder einem Wirkstoff in die Luft.

### HINTERGRUND UND STAND DER TECHNIK

Die Verschlechterung der Luftqualität, insbesondere in Innenräumen, ist ein Problem, das in den kommenden Jahren noch zunehmen dürfte, beispielsweise in Niedrigenergiehäusern, in denen ein regelmäßiger Raumluftaustausch unerwünscht ist. Um Energieverbrauch und -verlust in Gebäuden zu minimieren, werden die Häuser heute luftdicht gebaut. Durch diese Bauweise wird ein stetiger Luftaustausch, den es noch vor einigen Jahren durch undichte Fenster und Türen gab, eliminiert. Stattdessen müssen die Häuser heute gelüftet oder mit teuren Lüftungsanlagen ausgestattet werden. Eine temporäre Verwendung der hier offenbarten Erfindung, besonders in Schlafräumen und besonders bei sehr kaltem oder heißem Wetter, bietet eine effektive Verbesserung der Lebensqualität der betreffenden Menschen.

Die Weltgesundheitsorganisation (WHO) schätzt, dass weltweit jedes Jahr etwa sieben Millionen Menschen vorzeitig an Krankheiten sterben, die auf Luftverschmutzung zurückzuführen sind. Schlechte Luftqualität wird mit einer Reihe von Krankheiten in Verbindung gebracht, darunter Asthma, Lungenentzündung, Lungenkrebs, chronisch obstruktive Lungenerkrankungen und Herz-Kreislauf-Erkrankungen. Dies deutet darauf hin, dass das Einatmen von verschmutzter Luft fast so schädlich ist wie etwa das Rauchen. Nach Angaben der US-Umweltschutzbehörde (EPA) ist die Luftverschmutzung in Innenräumen in der Regel 2- bis 5-mal so hoch wie im Freien. Da viele Großstädte in Entwicklungsländern Schwierigkeiten haben, die Verschlechterung der Luftqualität am Ursprung zu verhindern, wurden verschiedene Lösungen entwickelt, um die Luftqualität in Innenräumen zu verbessern.

Eine bekannte Methode zur Verbesserung der Raumluft ist die Verwendung von Lufterfrischungsartikeln, die Düfte in die Luft sprühen. Solche Produkte können zwar das Vorhandensein von Schadstoffen in der Luft verschleiern und einen angenehmen Eindruck bieten, doch ist bekannt, dass sie den Gehalt an flüchtigen organischen Bestandteilen erhöhen, was bei übermäßigem Gebrauch zu einer Verschlechterung der Luftqualität führt. Solche Geräte können auch so programmiert werden, dass sie nach einem bestimmten Zeitplan Duft auslösen und somit kein aktives Eingreifen seitens des Benutzers benötigen. Das Ergebnis ist ein praktisches Gerät, das den oberflächlichen Eindruck einer verbesserten Luftqualität vermittelt.

Um die Luftqualität auf einer grundsätzlichen Ebene zu verbessern, wurden Luftreiniger entwickelt. Luftreiniger für Privathaushalte verwenden in der Regel folgende Techniken, um Partikel aus der Luft zu entfernen: Luftfilter mit Fasermaterial und/oder elektronische Luftreiniger (einschließlich elektrostatischer Abscheider und Ionisatoren). Fasermedien-Luftfilter entfernen Partikel, indem sie diese auf faserigen Filtermaterialien festhalten. Elektronische Luftreiniger entfernen Partikel durch ein aktives elektrostatisches Aufladeverfahren, das Elektrizität einsetzt, um Partikel aufzuladen, die von entgegengesetzt geladenen Platten angezogen werden und daran haften. Diese Geräte können zwar die Anzahl der schädlichen Partikel in der Luft verringern, haben aber keinen Einfluss auf die Gaszusammensetzung oder die wahrgenommene Luftqualität.

Wie eine Studie der US-Umweltbehörde EPA zeigt, werden Luftreiniger nach dem Kauf oft nicht mehr benutzt und die Geräte werden vernachlässigt. Dies ist häufig auf den Geräuschpegel der Geräte zurückzuführen. Ein weiterer Grund für die mangelnde Nutzung könnte die fehlende Wahrnehmung der Wirkung des Luftreinigers sein, da die meisten Partikel für Sinnesorgane nicht vollständig wahrnehmbar sind. Die Wirksamkeit der Geräte wird somit von dem Benutzer nicht bemerkt. Es besteht daher Bedarf an einem Gerät zur Verbesserung der Luftqualität in Innenräumen, das dem Benutzer eine positivere Erfahrung vermittelt, sodass er das Gerät konsequent benutzet und wartet.

Andererseits sind Freizeitgeräte bekannt, die sowohl zur Freisetzung von Aromen als auch zur Nachahmung des Rauchens verwendet werden. Diese können verwendet werden, um ein Gas- oder Aerosolgemisch freizusetzen, das einen aromatisierten oder parfümierten Stoff und einen Wirkstoff wie Kannabinol enthält. Vor allem für Nutzer, die früher traditionelle Produkte geraucht haben, ist die Verwendung dieser Geräte eine angenehme Erfahrung, und statt unerwünschte Rauchgerüche im Raum zu hinterlassen, entsteht dank des Dufts der Eindruck einer sauberen Luftqualität.

US2016325055A1 offenbart ein Beispiel eines solchen Geräts. Das Gerät ist so konfiguriert, dass es eine anpassbare Mischung aus mehreren Aromen und/oder Wirkstoffen entweder zur direkten Inhalation oder zur Freisetzung in einen Raum vaporisiert. Alternativ können die von dem Gerät freigesetzten Dämpfe auch zur Erholung oder für Wellnesszwecke verwendet werden. Die Komponenten der Mischung werden getrennt gelagert und je nach gewünschter Rezeptur im Gerät gemischt. Diese Komponenten können in Form eines komprimierten Gases, einer Flüssigkeit, insbesondere einer unter Druck gelagerten Flüssigkeit, oder eines Feststoffs vorliegen. Druckluft kann als Hilfsmittel verwendet werden, um ein flüssiges Gemisch in ein Aerosol zu verwandeln.

Das Gerät gemäß US2016325055A1 kann zwar eine bessere Benutzererfahrung bieten, die zu einem gewohnheitsmäßigen Gebrauch führt, aber sein Prinzip der Luftverbesserung ähnelt dem eines Lufterfrischers. Es werden dem Luft Stoffe hinzugefügt, die einen guten sensorischen Eindruck vermitteln, aber die Luftqualität aus gesundheitlicher Sicht nicht verbessern.

Es besteht daher ein Bedarf an einem Luftanreicherungsgerät, das die Qualität der eingeatmeten Luft verbessert und gleichzeitig einfach und angenehm zu bedienen ist.

### AUFGABE DER ERFINDUNG

Aufgabe der Erfindung ist es, eine Vorrichtung für die Anreicherung von Luft mit Sauerstoff, ohne die Nachteile des Standes der Technik bereitzustellen.

Insbesondere war es eine Aufgabe der Erfindung, eine Vorrichtung für die Anreicherung von Luft, die in erster Linie die Luftzusammensetzung verbessert, aber auch eine sekundäre Funktion erfüllt, die dem Benutzer ein angenehmes sensorisches Erlebnis bietet, bereitzustellen. Zudem war es eine Aufgabe der Erfindung eine Vorrichtung bereitzustellen, welche eine "gesunde Alternative" zu den E-Zigaretten darstellt. Eine weitere Aufgabe der Erfindung war es, eine Vorrichtung für die Anreicherung von Luft als Inhalationsartikel oder als Lufterfrischungsartikel bereitzustellen.

### ZUSAMMENFASSUNG DER ERFINDUNG

Gelöst wird die Aufgabe durch die Merkmale der unabhängigen Ansprüche. Vorteilhafte Ausgestaltungen der Erfindung sind in den abhängigen Ansprüchen und in der Beschreibung offenbart.

In einem ersten Aspekt betrifft die Erfindung eine Vorrichtung für die Anreicherung von Luft umfassend eine Steuereinheit und mindestens einen Sensor. Die Vorrichtung umfasst weiterhin eine erste Kammer und eine zweite Kammer, wobei die erste Kammer dafür konfiguriert ist, Sauerstoff unter Druck zu halten und als Reaktion auf ein erstes Signal von der Steuereinheit den Sauerstoff mit einem ersten Volumenstrom abzugeben. Die zweite Kammer ist dafür konfiguriert, als Reaktion auf ein zweites Signal von der Steuereinheit einen inhalierbaren Dampf oder Aerosol mit einem zweiten Volumenstrom abzugeben, wobei der erste Volumenstrom größer als der zweite Volumenstrom ist.

Der inhalierbare Dampf oder das Aerosol enthält vorzugsweise einen Hilfsstoff. Durch Lagerung des Sauerstoffs und des Hilfsstoffs in getrennten Kammern können die beiden Flüssigkeiten in situ angemischt werden, um eine bestimmte Zusammensetzung zu erhalten, die mehr Sauerstoff als Hilfsstoff enthält. Die Trennung der Kammern ermöglicht auch die Freisetzung von Sauerstoff unabhängig von dem Hilfsstoff. Die Verwendung getrennter Kammern ermöglicht auch einen modularen Einsatz der Vorrichtung, bei dem eine Komponente unabhängig von der anderen nachgefüllt oder ausgetauscht werden kann. So kann die Vorrichtung beispielsweise eine große Kammer mit Sauerstoff für eine längerfristige Verwendung und eine kleinere Kammer für ein häufig zu wechselndes Aroma umfassen. Diese Trennung der Kammern ermöglicht auch eine überraschend wirtschaftlichere Nutzung und Wartung.

Durch die Freisetzung eines größeren Volumens an Sauerstoff als an Hilfsstoffen weist der aus der Vorrichtung austretende Strom einen höheren Anteil an Sauerstoff auf. Der Sauerstoff dient also nicht nur zur Unterstützung der Dispersion des Hilfsstoffs, sondern ist der Hauptbestandteil, der aus der Vorrichtung freigesetzt wird. Die primäre Funktion der Vorrichtung ist also die Erhöhung des Sauerstoffgehalts in der eingeatmeten Luft, während das Vorhandensein des Hilfsstoffs eine sekundäre Rolle spielt. Da das erhöhte Vorhandensein von Sauerstoff für den Benutzer möglicherweise nicht wahrnehmbar ist, kann das aus der zweiten Kammer freigesetzte Material eine sensorische Funktion erfüllen. Diese kann insbesondere visuell, olfaktorisch oder gustatorisch sein. Vorzugsweise ist der Hilfsstoff so konfiguriert, dass er dem Benutzer ein positives sensorisches Feedback gibt, was im Gegensatz zu den duftneutralen und lärmenden Geräten des Standes der Technik die Wahrscheinlichkeit erhöht, dass die Vorrichtung konsequent und effektiv eingesetzt wird. Die erfindungsgemäße Vorrichtung wird somit in der Praxis einen größeren Beitrag zur Verbesserung der Luftqualität als die bekannten Vorrichtungen leisten. Dies löst die erfindungsgemäße Aufgabe überraschend gut.

Aufgrund des Vorhandenseins mindestens eines Sensors in der Vorrichtung kann diese so konfiguriert werden, dass sie bei Bedarf Sauerstoff abgibt. Die Vorrichtung kann beispielsweise den Sauerstoff nur abgeben, wenn der Sauerstoffgehalt in der Umgebung unter eine Untergrenze sinkt oder wenn die Abgabe von Sauerstoff vom Benutzer ausgelöst wird, z.B. wenn der Benutzer aus der Vorrichtung inhaliert. Die Funktion der Vorrichtung ist somit zumindest teilweise automatisiert. Dies verbessert überraschenderweise das Benutzererlebnis und führt zu einer häufigeren Nutzung der Vorrichtung.

Mittels des Sensors und der Steuereinheit ist der von der Vorrichtung abgegebene Strom vorteilhafterweise anpassbar. Der Sensor und die Steuereinheit können vorzugsweise die Menge und/oder den Anteil des freigesetzten Sauerstoffs entsprechend dem vom Sensor festgestellten Bedarf anpassen. Es kann insbesondere bevorzugt sein, dass die Steuereinheit der erfindungsgemäßen Vorrichtung Daten von einem Sensor für die Luftsauerstoffkonzentration in der Umgebung verwendet, um die Durchflussrate und/oder die Konzentration von Sauerstoff in dem von der Vorrichtung abgegebenen Strom einzustellen. Vorzugsweise sind die Durchflussrate und die Konzentration des freigesetzten Sauerstoffs so ausgelegt, dass die Sauerstoffkonzentration in der Umgebung bei mindestens 21 vol.%, vorzugsweise bei mindestens 25 vol.% liegt. Die Vorrichtung ist somit überraschend reaktiv und dynamisch, mit minimalen Eingaben des Benutzers.

In einer bevorzugten Ausführungsform der Erfindung, sollte der Anteil an Sauerstoff in einem von der Vorrichtung abgegebenen Strom mindestens 25%, mindestens 30%, mindestens 40%, mindestens 50%, mindestens 60%, mindestens 70%, mindestens 80% oder mindestens 90% des gesamten Volumens des Stroms betragen, je nach individuellem Empfinden des Benutzers und je nachdem, ob das Gerät für die direkte oder indirekte Inhalation konfiguriert ist.

Bei Vorrichtungen zur direkten Inhalation (d.h. vorzugsweise Vorrichtung gemäß der Erfindung, die mit einem Mundstück vorgesehen sind) beträgt der volumetrische Sauerstoffanteil in einem Strom, der aus dem Mundstück austritt, vorzugsweise mindestens 25%. Dieser Strom kann eine Kombination aus sauerstoffangereicherter Luft aus der ersten Kammer (Sauerstoffkammer), einem Luft- oder Wirkstoff aus der zweiten Kammer sowie einer eventuellen Druckluft umfassen, die zur Freisetzung des Luft- oder Wirkstoffs verwendet werden kann, sowie jeden anderen Luftstrom, der durch das Mundstück strömen darf. Bei diesem Prozentsatz kann die Inhalation des sauerstoffangereicherten Stroms die Müdigkeit verringern und die allgemeine Gesundheit verbessern, während sie im Allgemeinen für alle Bevölkerungsgruppen sicher ist.

Vorzugsweise sollte der volumetrische Sauerstoffanteil, der das Mundstück verlässt, mindestens 30% betragen. Studien haben gezeigt, dass Sauerstoffkonzentrationen von mindestens 30 % die Müdigkeit gesunder Erwachsener verringern und die Reaktionszeiten und die Ausdauer beim Fahren verbessern (Sung et al, Effects of oxygen concentrations on driver fatigue during simulated driving, Applied Ergonomics, Volume 36, Issue 1, 2005, Pages 25-31, ISSN 0003-6870, https://doi.org/10.1016/j.apergo.2004.09.003). Studien haben auch gezeigt, dass Sauerstoffkonzentrationen von mindestens 30% die kognitiven Fähigkeiten gesunder Studenten verbessern (Chung et al., Effect of 30% oxygen administration on verbal cognitive performance, blood oxygen saturation and heart rate. Appl Psychophysiol Biofeedback. 2006 Dec;31(4):281-93. Doi: 10.1007/s10484-006-9023-5. PMID: 17053947). Weitere Studien haben gezeigt, dass eine Sauerstoffkonzentration von 30% das Gedächtnis, das räumliche Vorstellungsvermögen, die Herzgesundheit und die geistige Gesundheit verbessert, um nur einige Beispiele zu nennen.

Ein volumetrischer Sauerstoffanteil von mindestens 40 % kann ebenfalls von Vorteil sein. Die Leistung von Probanden bei mathematischen Aufgaben verbesserte sich, nachdem sie Luft mit einer Sauerstoffkonzentration von 40% erhalten hatten (Chung et al., A study on the effects of 40% oxygen on addition task performance in three levels of difficulty and physiological signals. Int J Neurosci. 2008 Jul;118(7):905-16. doi: 10.1080/00207450701750455. PMID: 18569150*.).* Bei dieser Konzentration sind weitere Vorteile in Bezug auf Benutzererfahrung, gesundheitliche Wirkung und Verbesserung der Luftqualität zu erwarten.

Für bestimmte Anwendungen können Vorrichtungen, die für die direkte Inhalation konfiguriert sind, vorzugsweise einen Strom mit einem volumetrischen Sauerstoffanteil von mindestens 50%, mindestens 60% oder mehr abgeben. Solche Ausführungsformen können auf bestimmte Bevölkerungsgruppen ausgerichtet sein und/oder nur auf ärztlichen Rat hin verwendet werden. Vorzugsweise überschreitet der volumetrische Sauerstoffanteil in Ausführungsformen der Erfindung, die für die direkte Inhalation ausgelegt sind, nicht 65%.

Andererseits können bei Vorrichtungen gemäß Ausführungsformen der Erfindung, die für die Abgabe eines Gases oder Aerosols in einen Raum ("Raumgeräte") konfiguriert sind, höhere volumetrische Anteile von Sauerstoff bevorzugt sein. Solche Vorrichtungen können vorzugsweise Sauerstoff in einem volumetrischen Anteil von mindestens 60%, mindestens 70%, noch bevorzugter mindestens 80% und noch bevorzugter mindestens 90% freisetzen. Besonders bevorzugt umfasst der Anteil an Sauerstoff mindestens 95 Volumenprozent. Bei Raumgeräten ist es bevorzugt, dass der Anteil an Sauerstoff immer nahezu 100% beträgt. Die Volumenanteile sind bei Umgebungstemperatur und -druck zu betrachten, vorzugsweise bei 25 °C und 1 atm. Bei diesen Verhältnissen wird die Vorrichtung insgesamt zu einer Verbesserung der Luftqualität führen. Alle zusätzlichen Effekte, die sich aus den Hilfsstoffen ergeben, können eine untergeordnete Rolle spielen, werden aber die Luftqualität insgesamt nicht verringern. Es war überraschend, dass selbst der kleine Anteil an Hilfsstoff ausreichte, um dem Benutzer einen positiven Sinneseindruck zu vermitteln, sodass die Luftqualität regelmäßig verbessert wird. Mit anderen Worten, der geringe Anteil an Duft oder Wirkstoff wurde als ausreichend befunden, um die Funktion der Vorrichtung wahrnehmbar und spürbar zu machen. Dies steht im Gegensatz zu den bekannten luftreinigenden Geräten. Darüber hinaus kann die erfindungsgemäße Vorrichtung hiermit auch als eine gesündere Alternative zu einigen bekannten Vorrichtungen wie Lufterfrischern (auf Basis von flüchtigen organischen Chemikalien) und E-Zigaretten fungieren. Dies löst die Aufgabe der Erfindung besonders gut.Die Anteile des freigesetzten Sauerstoffs und Hilfsstoffs sowie deren Durchflussraten müssen unter Berücksichtigung des Anwendungsfalls der Vorrichtung und etwaiger Sicherheitsgrenzen begrenzt werden. Zum Beispiel werden hohe Durchflussraten und hohe Sauerstoffanteile in entflammbaren Umgebungen nicht empfohlen. Falls die Vorrichtung für die direkte Inhalation konfiguriert ist, sind die biologischen Sicherheitsgrenzwerte hinsichtlich der Flussrate und des Anteils von Sauerstoff und Hilfsstoffen zu beachten.

In einer bevorzugten Ausführungsform der Erfindung umfasst die erste Kammer ein mit Sauerstoff angereichertes Gas, insbesondere mit Sauerstoff angereicherte Luft. Vorzugsweise wird der Sauerstoff in der ersten Kammer in einem Volumenanteil von 95% vorhanden sein und kann mittels eines Drucksensors beim Inhalieren individuell reguliert werden.

In einer bevorzugten Ausführungsform der Erfindung ist die zweite Kammer dafür konfiguriert, einen Wirkstoff und/oder einen Duft zu lagern, wobei der Wirkstoff und/oder der Duft vorzugsweise in flüssiger Form gelagert sind. Durch die Aufnahme eines Wirkstoffs oder eines Aromas in das Hilfsmaterial kann ein Benutzer wahrnehmen, dass die Vorrichtung aktiv ist, und die Vorrichtung als wirksamer als herkömmliche Geräte zur Verbesserung der Luftqualität empfinden. Anders als bei herkömmlichen Lufterfrischungsgeräten sorgt die Freisetzung von Sauerstoff zusammen mit dem Wirkstoff oder Duft dafür, dass der Gesamteffekt des Geräts auf die Luftqualität immer positiv ist. Aufgrund des hohen Sauerstoffanteils kann die Vorrichtung eine geeignete Alternative für Benutzer sein, die mit dem Rauchen oder Dampfen aufhören wollen, aber das Ritual des Inhalierens aus einer Vorrichtung noch nicht aufgeben wollen. Die Vorrichtung ist auch eine überraschend gute Alternative für Benutzer, die den Geruch von Innenräumen verbessern oder eine entspannende Atmosphäre schaffen wollen.

Überraschenderweise ist die Vorrichtung auch für solche Benutzer nützlich, die Schwierigkeiten haben, genügend Sauerstoff in ihren Blutkreislauf zu bekommen, z. B. aufgrund der langfristigen Auswirkungen von Covid oder einer anderen Erkrankung. Vor allem bei Personen, die keine fachärztliche Betreuung benötigen, kann der Alltag mit solchen Erkrankungen zu Müdigkeit und Atemnot führen. Die Vorrichtung bietet eine überraschend effektive und benutzerfreundliche Möglichkeit, solche Symptome durch verbesserte Atemluft zu lindern.

Die Aufbewahrung des Wirkstoffs oder des Dufts in flüssiger Form, z. B. gelöst in Propylenglykol oder pflanzlichem Glycerin, ermöglicht eine längere Nutzung der zweiten Kammer, bevor ein Nachfüllen oder ein Austausch erforderlich ist. Da eine Flüssigkeit nicht unter sehr hohem Druck gelagert werden muss und bei Verdampfung oder Vernebelung ein großes Gasvolumen erzeugen kann, ist die Vorrichtung sicher und wartungsarm. Durch die Bereitstellung des Wirkstoffs oder Duftes in flüssiger Form konnte die Vorrichtung überraschend kompakt gestaltet werden.

In einer weiteren bevorzugten Ausführungsform der Erfindung umfasst die Vorrichtung einen Auslass, wobei der Auslass mit der ersten Kammer in Fluidverbindung steht, vorzugsweise mittels eines ersten Ventils, und der Auslass mit der zweiten Kammer in Fluidverbindung steht, vorzugsweise mittels eines zweiten Ventils. Die Verwendung von Ventilen, die jede Kammer mit dem Auslass verbinden, ermöglicht eine unabhängige Freigabe oder Vermischung der Komponenten aus den beiden Kammern. Die Ventile können verwendet werden, um eine periodische, kontinuierliche oder bedarfsabhängige Freisetzung von Gas aus der Vorrichtung zu gewährleisten und gleichzeitig die Kammern bei Inaktivität abzudichten. Durch die Verwendung von Ventilen zur selektiven Freigabe der Flüssigkeiten und/oder Gasen kann die Vorrichtung überraschend lange halten, ohne dass die Kammer gewechselt oder nachgefüllt werden müssen.

In einigen bevorzugten Ausführungsformern der Erfindung sich das erste Ventil und das zweite Ventil identisch.

In einer bevorzugten Ausführungsform der Erfindung sind das erste Ventil und das zweite Ventil Magnetventile. Ein Magnetventil kann durch einen Elektromagneten als Beispiel für einen Aktuator betrieben werden und kann sehr schnell auf die Betätigung reagieren, was zu einer reibungsloseren Nutzung der Vorrichtung führt. Außerdem können Magnetventile hohen Drücken standhalten und sind daher besonders für den Einsatz mit komprimierten Gasen geeignet.

In einer weiteren bevorzugten Ausführungsform der Erfindung ist die Steuereinheit so konfiguriert, dass sie das erste und/oder das zweite Ventil in Reaktion auf Signale von dem mindestens einen Sensor öffnet und/oder schließt. Die Steuereinheit kann alternativ oder zusätzlich die Ventile nur teilweise öffnen, und zwar in einem Umfang, der durch die erfassten Signale und eine vorgegebene Endzusammensetzung bestimmt wird. Dies ermöglicht eine überraschend flexible Nutzung der Vorrichtung.

Der Begriff "Signal" bezeichnet hier vorzugsweise einen elektrischen Strom, dessen Stromstärke, Spannung oder Widerstand Rückschlüsse auf das Vorhandensein eines Umstandes oder die Größe eines Parameters zulässt.

Der Begriff "Steuereinheit" bezieht sich hier vorzugsweise auf eine beliebige Rechnereinheit mit einem Prozessor, einem Prozessorchip, einem Mikroprozessor oder einem Mikrocontroller, die eine automatische Steuerung der Komponenten des Geräts ermöglicht, z. B. die Ventile und/oder mögliche Aktuatoren wie einen Motor oder Elektromagnet zur Einstellung des Öffnungsgrads eines Ventils. Die Komponenten der Steuereinheit können konventionell oder individuell für die jeweilige Implementierung konfiguriert sein. Vorzugsweise umfasst die Steuereinheit einen Prozessor, einen Speicher und einen Computercode (Software/Firmware) zur Steuerung der Komponenten der Vorrichtung.

Die Steuereinheit kann auch eine programmierbare Leiterplatte, einen Mikrocontroller oder eine andere Vorrichtung zum Empfangen und Verarbeiten von Datensignalen von den Komponenten der Vorrichtung umfassen, beispielsweise von Sensoren wie beispielsweise Drucksensoren, Sauerstoffsensoren, und/oder Wärmesensoren.

Die Steuereinheit umfasst vorzugsweise ferner ein computerverwendbares oder computerlesbares Medium, wie eine Festplatte, einen Direktzugriffsspeicher (RAM), einen Festwertspeicher (ROM) oder einen Flash-Speicher., auf dem eine Computersoftware oder ein Code installiert ist. Der Computercode oder die Software zur Steuerung der Komponenten des Geräts kann in einer beliebigen Programmiersprache oder einer modellbasierten Entwicklungsumgebung geschrieben werden, z. B. in C/C++, C#, Objective-C, Java, Basic/VisualBasic, MATLAB, Python, Simulink, StateFlow, Lab View oder Assembler.

Die Software und alle funktionalen Beschreibungen der Software durch die Beschreibung der Steuerung bestimmter Komponenten oder Aspekte des hierin beschriebenen Geräts werden als technische Merkmale betrachtet, da sie eine direkte physische Ausgabe auf der Vorrichtung darstellen. Funktionsbeschreibungen von Software können daher als bevorzugte und definierende Ausführungsformen der Erfindung angesehen werden. Der jeweils verwendete Computercode steht dem Fachmann zur Verfügung und kann nach dem Stand der Technik entsprechend konstruiert werden.

Der Begriff "Steuereinheit ist konfiguriert, um" einen bestimmten Arbeitsschritt auszuführen, wie z.B. ein Ventil zu öffnen und/oder zu schließen, kann eine kundenspezifische oder standardmäßige Software umfassen, die auf der Steuereinheit installiert ist und diese Betriebsschritte initiiert und regelt.

In einer weiteren bevorzugten Ausführungsform der Erfindung umfasst die zweite Kammer einen Verdampfer für die Verdampfung der zweiten Flüssigkeit. Der Verdampfer kann sehr kompakt sein und die Flüssigkeit in eine Gasform umwandeln, die sicher eingeatmet werden kann. Alternativ kann die Flüssigkeit auch aerosoliert werden, wobei ein Teil der Flüssigkeit zu Gas umgewandelt wird und ein Teil der Flüssigkeit als winzige Tröpfchen in dem Gas suspendiert bleibt. Durch die zusätzliche Erwärmung des Gases kann dieses tendenziell nach oben in den Raum abgegeben werden und sich überraschend leicht im freien Raum ausbreiten.

In einer weiteren bevorzugten Ausführungsform der Erfindung umfasst die Vorrichtung einen Verdampfer für die Verdampfung der zweiten Flüssigkeit, wobei der Verdampfer durch eine Trennwand von der zweiten Kammer getrennt vorliegt. Der Verdampfer kann zum Beispiel eine Heizspirale oder ein Heizelement umfassen. Je nach Natur der Flüssigkeit kann es vorteilhaft sein, den Verdampfer nicht direkt mit der Flüssigkeit in Berührung kommen zu lassen, z. B. wenn die Flüssigkeit dazu neigt, den Verdampfer zu verschmutzen oder einen Rückstand darauf zu hinterlassen. Dadurch wird die Vorrichtung auch überraschend wartungsarm und die zweite Kammer kann überraschend leicht und günstig ohne den Verdampfer ausgetauscht werden.

Alternativ kann der Verdampfer auch in der zweiten Kammer untergebracht werden. Dies kann beispielsweise helfen, die Menge der verdampften Flüssigkeit zu begrenzen, indem eine kleine Menge Flüssigkeit über einen Docht zum Verdampfer geleitet wird. Die so ausgestaltete Vorrichtung ist überraschend effizient.

In einer weiteren bevorzugten Ausführungsform der Erfindung ist der mindestens eine Sensor ein Drucksensor. Der Drucksensor ist vorzugsweise dafür konfiguriert, das Ansaugen aus der Düse zu erfassen. Die Steuereinheit ist vorzugsweise so konfiguriert, dass sie das erste und das zweite Ventil öffnet, wenn ein Unterdruck erfasst wird. Der Drucksensor ist besonders geeignet, um zu erkennen, wann ein Benutzer die Vorrichtung aktivieren möchte, bevorzugt wenn diese als Inhalationsartikel konfiguriert ist. Der Gebrauch der Vorrichtung kann somit natürlich und reibungslos erfolgen, ähnlich wie der Gebrauch einer herkömmlichen Zigarette.

Vorzugsweise ist die Steuereinheit auch so konfiguriert, dass sie den Verdampfer aktiviert, wenn einen Unterdruck erkannt wird. Die Steuereinheit ist vorzugsweise so konfiguriert, dass sie das erste und zweite Ventil schließt und die Aktivierung des Verdampfers beendet, wenn kein Unterdruck mehr festgestellt wird. Die Benutzung der Vorrichtung kann somit weitgehend automatisch und bequem erfolgen und kann die Benutzung einer Zigarette imitieren.

In einer weiteren bevorzugten Ausführungsform der Erfindung ist der Auslass als Mundstück konfiguriert. Das Mundstück kann die Form einer Düse haben, die die Dispersion von großen Flüssigkeitströpfchen fördert. Das Mundstück kann auch einen Einwegfilter enthalten. Vorzugsweise ist das Mundstück vom Rest der Vorrichtung lösbar, sodass es leicht gereinigt oder ersetzt werden kann. Die Vorrichtung ist somit besonders hygienisch und kostengünstig.

In einer weiteren bevorzugten Ausführungsform der Erfindung ist der Auslass als Zerstäuber konfiguriert. Damit kann Gas oder ein Aerosolgemisch direkt in einen Raum gesprüht werden, z. B. in ein Zimmer. Vorteilhafterweise kann ein Zerstäuber einen Verdampfer überflüssig machen, da die Flüssigkeit einfach mechanisch in kleine Tröpfchen zerstäubt werden kann, die sich leicht in der Umgebungsluft verteilen. Der Stromverbrauch der Vorrichtung kann dadurch überraschend stark reduziert werden. Ein Akkumulator oder Batterie muss daher seltener gewechselt oder aufgeladen werden, was den Wartungsaufwand für den Benutzer verringert.

In einer weiteren bevorzugten Ausführungsform der Erfindung umfasst die Vorrichtung einen Akkumulator. Vorzugsweise ist dies Teil einer Baueinheit, die leicht entfernt und wieder in die Vorrichtung eingebaut werden kann. Die Baueinheit kann optional auch die Steuereinheit und einen oder mehrere Sensoren umfassen. Der Akkumulator ermöglicht die Tragbarkeit der Vorrichtung, z. B. um den Transport der Vorrichtung von Raum zu Raum zu erleichtern oder um es unterwegs z. B. als Inhalationsartikel zu verwenden. Vorzugsweise ist der Akkumulator wiederaufladbar, zum Beispiel über einen USB-Anschluss.

In einer weiteren bevorzugten Ausführungsform der Erfindung umfasst die Vorrichtung Mittel zum Anschluss der Vorrichtung an das Stromnetz. Dies kann zum Beispiel ein Stecker sein. Hierdurch kann die Vorrichtung mit höheren Stromstärken betrieben werden, die einen kontinuierlichen Einsatz einer größeren Vorrichtung ermöglichen, beispielsweise zur regelmäßigen Überwachung und Verbesserung der Luftqualität in einem großen Raum.

In einer weiteren bevorzugten Ausführungsform der Erfindung umfasst die Vorrichtung weiterhin einen Sauerstoffsensor, wobei die Steuereinheit dafür konfiguriert ist, das erste Ventil zu öffnen, wenn ein ermittelter Sauerstoffgehalt unter ein vorgegebenes Niveau fällt. Ein Sauerstoffsensor kann vorzugsweise in Kontakt mit der Umgebungsluft sein und durch Signale mit der Steuereinheit kommunizieren. Die Verwendung der Vorrichtung kann somit weiter automatisiert werden und der Sauerstoff kann unabhängig vom Vorhandensein ausreichender Hilfsstoffe freigesetzt werden. Dies ist besonders nützlich für Benutzer mit Atembeschwerden, da ein Absinken des Sauerstoffgehalts in der Umgebung mit den Sinnen oft nicht wahrnehmbar ist.

Darüber hinaus kann die Steuereinheit so konfiguriert sein, dass sie einen optischen oder akustischen Alarm auslöst, wenn der Sauerstoffgehalt unter einen kritischen Schwellenwert fällt und/oder wenn der Sauerstoffgehalt in der ersten Kammer unter einen kritischen Wert fällt. Dies kann vorzugsweise durch eine oder mehrere LEDs, Lautsprecher an einem Gehäuse der Vorrichtung und/oder Drucksensoren oder Partialdrucksensoren in der ersten Kammer erfolgen.

In einer weiteren bevorzugten Ausführungsform der Erfindung umfasst die Vorrichtung ferner einen Mischkanal, der sowohl mit der ersten und der zweiten Kammer als auch mit dem Auslass in Fluidverbindung steht. Der Mischkanal kann verwendet werden, um sicherzustellen, dass der inhalierte Strom die gewünschte Zusammensetzung hat. Der Mischkanal kann auch so konfiguriert sein, dass eine Flüssigkeit, die die zweite Kammer verlässt, aerosoliert wird, z. B. durch den hohen Druck des durch die Flüssigkeit strömenden Sauerstoffs.

In einer weiteren bevorzugten Ausführungsform der Erfindung umfasst die Vorrichtung eine dritte Kammer mit einer optionalen Komponente des inhalierbaren Gemisches. Die dritte Kammer umfasst vorzugsweise ein drittes Ventil. Die zweite Kammer kann ein Aroma enthalten, während die dritte Kammer einen Wirkstoff enthält. Alternativ können die zweite und die dritte Kammer unterschiedliche Komponenten enthalten, die zum Mischen eines Aromas verwendet werden. Die Steuereinheit ist vorzugsweise so konfiguriert, dass sie eine Rezeptur aus einem Speicher ausliest und die Komponenten entsprechend durch Öffnung der Ventile mischt. Die Vorrichtung umfasst vorzugsweise eine Schnittstelle, wie z. B. einen Drehknopf, der es dem Benutzer ermöglicht, die von der Vorrichtung ausgegebene Rezeptur zu ändern oder einzustellen.

Ein weiterer Aspekt der Erfindung betrifft die Verwendung einer Vorrichtung wie oben beschrieben zur Inhalation von Sauerstoff und/oder zur Verbesserung der Luftqualität in einem Raum.

In einem weiteren Aspekt betrifft die Erfindung einen Vorrichtungshauptkörper, der eine Steuereinheit, ein erstes Ventil und ein zweites Ventil wie oben beschrieben, Aufnahmemittel für einen Akkumulator, Aufnahmemittel für eine mit dem ersten Ventil fluidisch zu verbindende erste Kammer und Aufnahmemittel für eine mit dem zweiten Ventil fluidisch zu verbindende zweite Kammer, Aufnahmemittel für ein Mundstück oder einen Zerstäuber umfasst. Die Aufnahmemittel können zum Beispiel einen Gewindehals oder einen Schnappverschluss umfassen.

In einem weiteren Aspekt betrifft die Erfindung einen Bausatz, der einen Akkumulator und/oder einen Stromnetzanschluss, eine erste Kammer mit komprimiertem Sauerstoff, eine zweite Kammer mit einem Hilfsstoff, eine Steuereinheit, mindestens einen Sensor und ein Mundstück oder einen Zerstäuber umfasst. In einer bevorzugten Ausführungsform umfasst der Bausatz weiterhin ein Gehäuse, wobei der Akkumulator und/oder Stromnetzanschluss, einen optionalen Transformator, die erste Kammer, die zweite Kammer, die Steuereinheit, der Sensor, das Mundstück oder der Zerstäuber so gestaltet sind, dass sie mit Aufnahmemitteln des Gehäuses in Eingriff treten können.

### KURZBESCHREIBUNG DER ABBILDUNGEN

Um verschiedene bevorzugte Ausführungsformen der Erfindung zu veranschaulichen, werden die folgenden Figuren bereitgestellt. Eine detaillierte Beschreibung der bevorzugten Ausführungsformen folgt.
Fig. 1 zeigt eine schematische Darstellung einer batteriebetriebenen Vorrichtung gemäß einer ersten bevorzugten Ausführungsform der Erfindung.
Fig. 2 zeigt eine schematische Darstellung einer netzbetriebenen Vorrichtung gemäß einer zweiten bevorzugten Ausführungsform der Erfindung.
Fig. 3 zeigt eine schematische Darstellung einer netzbetriebenen Vorrichtung (oder Bausatz) gemäß einer dritten bevorzugten Ausführungsform der Erfindung.
Fig. 4 zeigt eine schematische Darstellung einer batteriebetriebenen Inhalationsvorrichtung gemäß einer vierten bevorzugten Ausführungsform der Erfindung.
Fig. 5 zeigt eine schematische Darstellung einer batteriebetriebenen Inhalationsvorrichtung gemäß einer fünften bevorzugten Ausführungsform der Erfindung.
Fig. 6 zeigt eine schematische Darstellung einer batteriebetriebenen Inhalationsvorrichtung gemäß einer sechsten bevorzugten Ausführungsform der Erfindung.

### DETAILLIERTE BESCHREIBUNG DER BEVORZUGTEN AUSFÜHRUNGSFORMEN

Im Folgenden soll die Erfindung anhand von Beispielen und Figuren näher erläutert werden, ohne auf diese beschränkt zu sein.

Die erfindungsgemäße Vorrichtung kann auf drei beispielhafte Arten konzipiert werden. Einerseits kann das Gerät als stehendes Haushaltsgerät konfiguriert werden, das ein inhalierbares Gas oder Aerosol in einen Innenraum wie eine Wohnung oder ein Büro abgibt. Die erste und die zweite bevorzugte Ausführungsform sind Beispiele für dieses Konzept. In einer weiteren Variante kann die erfindungsgemäße Vorrichtung als Erweiterungsmodul für bereits am Markt vorhandene Produkte, wie Klimaanlagen oder Raumluftreiniger, konzipiert werden. Die dritte bevorzugte Ausführungsform ist ein Beispiel hierfür. Andererseits kann die Vorrichtung als tragbarer Artikel konfiguriert werden, der das Gas oder Aerosol bei der Verwendung durch ein Mundstück zur direkten Inhalation freisetzt. Die vierte bis sechste bevorzugte Ausführungsform sind Beispiele für solche tragbaren Inhalationsartikel.

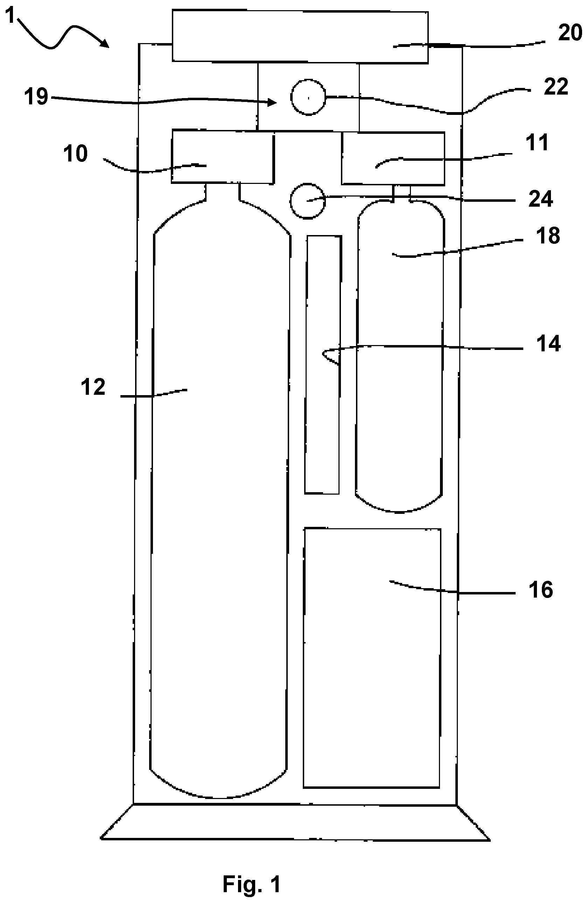

Die Fig. 1 zeigt schematisch eine Vorrichtung **1** gemäß der ersten bevorzugten Ausführungsform der Erfindung. Die Vorrichtung umfasst mindestens zwei Kammer. Eine erste Kammer **12** ist dafür konfiguriert, Sauerstoff unter Druck zu halten. Vorzugsweise ist die Kammer **12** ein Sauerstoffkapsel. Solche Kapsel können leicht von der Vorrichtung entfernt und gewechselt werden. Vorzugsweise ist die Kammer so konfiguriert, dass sie lösbar am Gerät befestigt werden kann, z. B. durch Verschraubung, Steckverbindung, Einrastung oder Schnappverschluss.

Eine zweite Kammer **18** ist dafür konfiguriert, einen Hilfsstoff zu halten. Der Hilfsstoff ist vorzugsweise ein Aroma oder ein Wirkstoff. Der Hilfsstoff kann in der zweiten Kammer als Flüssigkeit, komprimierte Flüssigkeit, komprimiertes Gas oder in jeder anderen verdampfbaren oder dispergierbaren Form gelagert werden. Das Hilfsmaterial wird vorzugsweise so ausgewählt, dass es dem Benutzer eine angenehme sensorische Rückmeldung gibt, z. B. durch Freisetzung eines angenehmen Geruchs. Alternativ oder zusätzlich kann der Hilfsstoff für aromatherapeutische Zwecke geeignet sein oder einen medizinischen oder erholsamen Wirkstoff enthalten. Der Hilfsstoff kann auch einen sichtbaren Dampf freisetzen, der z.B. durch eine oder mehrere LEDs beleuchtet wird. Die zweite Kammer liegt vorzugsweise in Form einer Kapsel vor. Analog wie für die erste Kammer ist die zweite Kammer vorzugsweise lösbar am Gerät befestigt.

Die erste Kammer ist mit einem ersten Ventil **10** verbunden, das so konfiguriert ist, dass es den Sauerstoff mit variablen Durchflussraten einstellbar freigibt. In dieser Ausführungsform ist der erste Ventil **10** ein Magnetventil. Die zweite Kammer ist mit einem zweiten Ventil **11** verbunden. Das zweite Ventil ist in diesem Fall ebenfalls ein Magnetventil und ist auch dafür konfiguriert, den Hilfsstoff mit einer einstellbaren Durchflussrate freizugeben. Vorzugsweise ist das zweite Ventil so konfiguriert, dass es den Hilfsstoff dispergiert oder aerosoliert, wenn es sich um eine Flüssigkeit handelt.

Zwischen den beiden Kammern und einem Auslass **22** befindet sich ein Mischventil **19**. Der Fluid aus der ersten Kammer **12** gelangt durch das erste Ventil **10** in das Mischventil **19**. Das erste Ventil **10** ist vorzugsweise durch einen Mischkanal mit dem Mischventil **19** verbunden. Fluid aus der zweiten Kammer **18** tritt durch das zweite Ventil **11** in das Mischventil **19** ein, vorzugsweise über den Mischkanal. Die Kammer kann so gestaltet sein, dass Druckverluste der Fluiden minimiert werden.

Ein Auslass **22** in Form eines Zerstäubers ist in Fluidverbindung mit dem Mischventil **19** vorgesehen. Der Auslass ist vorzugsweise so konfiguriert, dass er die Gasphase oder das aerosolierte Gemisch als breiten Sprühnebel abgibt und so die Vermischung mit der Umgebungsluft fördert. Der Auslass umfasst vorzugsweise eine Düse oder eine Venturi-Düse zur Erzeugung des Sprays. Ein Ein- und Ausschalter **20** ist weiterhin vorgesehen. Der Schalter **20** kann verwendet werden, um das Gas oder Aerosol mechanisch aus dem Auslass **22** freizugeben, oder er kann als Teil einer elektronischen Schaltung vorgesehen sein, die einen geeigneten Aktuator (z.B. ein Elektromagnet) aktiviert, um das Gas oder Aerosol aus dem Auslass **22** freizugeben. Vorzugsweise kann der Schalter **20** an der Oberseite der Vorrichtung angebracht sein und kann vom Benutzer nach unten gedrückt werden, um die Gasabgabe zu aktivieren. Dem Fachmann sind jedoch verschiedene alternative Aktivierungstechniken wie Fernsteuerung, App-Steuerung usw. bekannt, die in die Vorrichtung integriert werden können.

Die Zusammensetzung von Sauerstoff und Hilfsstoff wird so eingestellt, dass der volumetrische Anteil des Sauerstoffs größer ist als der volumetrische Anteil des gasförmigen oder aerosolierten Hilfsstoffs. Dementsprechend ist die zweite Kammer mindestens doppelt so klein wie das Volumen der ersten Kammer ausgelegt. Die Zusammensetzung des freigesetzten Gemisches wird vorzugsweise durch den Öffnungsgrad des ersten und zweiten Ventils eingestellt. Diese werden von einer Steuereinheit **14** gesteuert. Optional können die Ventile auch manuell steuerbar sein, z.B. über eine Spindel oder einen Flansch.

Es ist ein Sensor **24** zur Erfassung der Sauerstoffkonzentration in der Umgebungsluft vorgesehen. Bei dem Sensor handelt es sich vorzugsweise um einen Zusammensetzungssensor, der z. B. mit einer spektrometrischen Technik arbeitet. Der Sensor liefert periodisch oder kontinuierlich Signale an die Steuereinheit, die auf der erfassten Sauerstoffkonzentration basieren. Wenn der Sensor der Steuereinheit anzeigt, dass der Sauerstoffgehalt der Umgebungsluft unter einen vorbestimmten Schwellenwert gefallen ist, sendet die Steuereinheit **14** ein Signal an einen Aktuator, der das erste Ventil **10** öffnet. Vorzugsweise ist der Aktuator ein Motor oder ein Elektromagnet. Die Steuereinheit kann gleichzeitig auch das zweite Ventil **11** öffnen, so dass ein Gemisch aus Sauerstoff und Hilfsstoff in die Luft abgegeben wird. Vorzugsweise ist die Menge des Hilfsstoffs viel geringer als die des Sauerstoffs in dem Gemisch. Durch das Vorhandensein des Hilfsstoffs erhält der Benutzer eine sensorische Rückmeldung, die ihm mitteilt, dass das Gerät aktiv ist. Dies kann zu einer besseren Benutzererfahrung führen als bei Geräten, deren Wirkung für den Benutzer nicht sichtbar oder wahrnehmbar ist. Die Verbesserung der Luftqualität wird also spürbar gemacht.

In dieser bevorzugten Ausführungsform umfasst die Vorrichtung einen Akkumulator **16**. Vorzugsweise ist der Akkumulator **16** ebenfalls abnehmbar an der Vorrichtung befestigt.

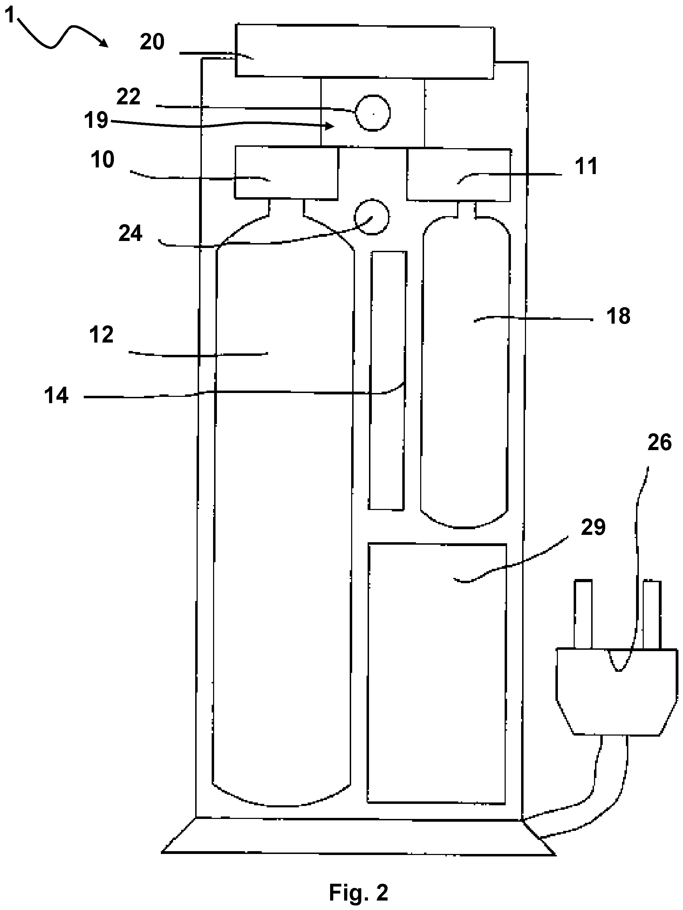

Die Figur 2 zeigt schematisch eine Vorrichtung gemäß der zweiten bevorzugten Ausführungsform der Erfindung. In dieser Ausführungsform ist die Vorrichtung für den Anschluss an das Stromnetz mittels einer Steckdose **26** konfiguriert. Die Vorrichtung umfasst optional auch einen Transformer **29** für die Anpassung der Spannung an die Bedürfnisse der Vorrichtung. Diese Vorrichtung ist vorzugsweise viel größer als die batteriebetriebene Vorrichtung der Fig. 1, wobei ihre Größe vergleichbar mit der Größe eines kleinen Schranks ist. Dies ermöglicht den Einsatz in größeren Räumen mit weniger häufigem Wechsel der Kapseln.

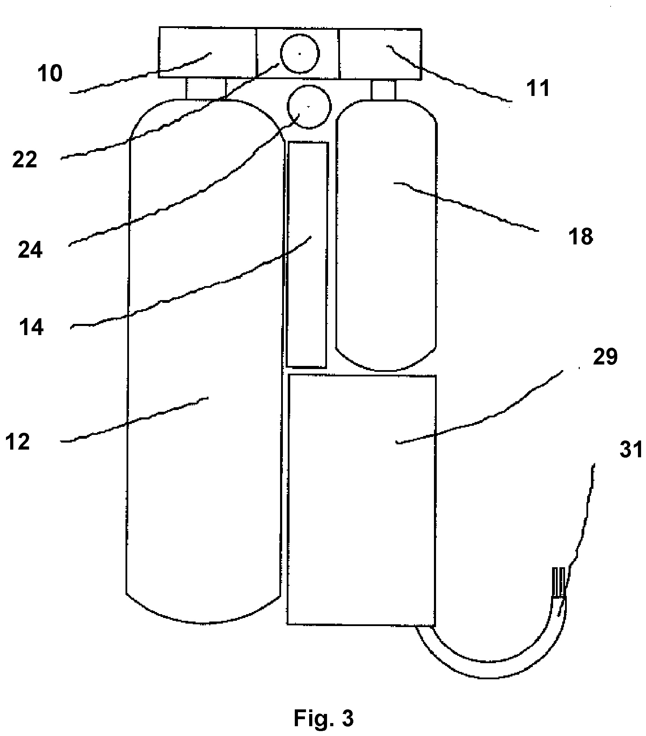

Die Figur 3 zeigt eine dritte bevorzugte Ausführungsform der Erfindung. Diese Ausführungsform umfasst im Wesentlichen die gleichen Komponenten wie in Fig. 1 und Fig. 2 beschrieben, ist jedoch für den Einsatz in einer bestehenden Vorrichtung wie einer Klimaanlage, einem Luftreiniger oder einem größeren Luftqualitätsmanagementsystem konfiguriert. Die Komponenten können durch ein Gehäuse miteinander verbunden sein, z.B. durch ein Grundgerüst, in das die Komponenten eingeklipst werden können, um sie in ihrer Position zu halten. In diesem Fall beschreibt diese Ausführungsform eine Vorrichtung, die in eine andere, größere Vorrichtung integriert und in deren Gehäuse untergebracht werden kann, um einen besseren ästhetischen Eindruck zu erzielen. Alternativ können die Komponenten auch ohne Gehäuse als Bausatz geliefert werden, den ein Benutzer in die bereits vorhandene Vorrichtung einbauen kann.

In dieser Ausführungsform sind eine erste Kammer **12**, die einen Sauerstoffbehälter umfasst, und eine zweite Kammer **18**, die einen Hilfsstoffbehälter umfasst, vorgesehen. Die erste Kammer **12** steht in Fluidverbindung mit einem ersten Ventil **10**. Die zweite Kammer **18** steht in Fluidverbindung mit einem zweiten Ventil **11**. Die beiden Ventile **10** und **11** stehen in Fluidverbindung mit einer Düse **22**, die als Auslass dient. Es ist nicht notwendig, dass die Ströme aus den beiden Kammern vor der Freigabe gemischt werden, so dass jeder separat zur Düse **22** fließen kann, ohne zuerst einen Mischkanal zu durchlaufen. Ein solcher Mischkanal kann jedoch optional vorgesehen werden.

Die Vorrichtung oder der Bausatz umfasst außerdem eine elektronische Steuereinheit **14**. Die Steuereinheit **14** kann dazu verwendet werden, das erste und das zweite Ventil **10**, **11** in Reaktion auf Signale zu steuern, die von einem Sensor, in diesem Fall einem Sauerstoffsensor **24**, erfasst werden. Dies ermöglicht eine vollautomatische Funktion ohne die Notwendigkeit eines manuellen Schalters. Da die Vorrichtung für den Benutzer im Wesentlichen unzugänglich sein kann, ist ein solcher Schalter möglicherweise nicht erwünscht. Die elektronischen Komponenten sind über einen Elektroanschluss **31** und einen Transformator **29**, der die Spannung des Stromnetzes in eine für die Vorrichtung geeignete Spannung umwandelt, an das Stromnetz angeschlossen. Dies ermöglicht die Integration der Vorrichtung in die Infrastruktur eines Gebäudes, insbesondere als Teil eines viel größeren Luftqualitätsmanagementsystems. Alternative Stromversorgungen wie z.B. über eine Batterie oder eine Steckdose sind ebenfalls denkbar.

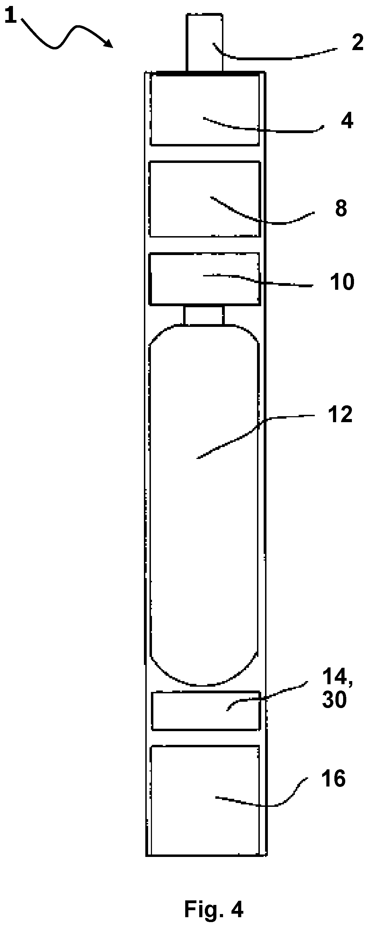

Die Figur 4 zeigt schematisch eine Vorrichtung **1** gemäß der vierten bevorzugten Ausführungsform der Erfindung. Die Vorrichtung **1** ist als Inhalationsartikel gestaltet. Die verschiedenen Komponenten sind im Wesentlichen nacheinander angeordnet, um die Vorrichtung eine schlanke äußerliche Form zu geben, welche beispielsweise bequem in einer Hosentasche passt. Die Vorrichtung umfasst ein Mundstück **2**, eine zweite Kammer **4**, einen Verdampfer **8**, ein erstes Ventil **10**, eine erste Kammer **12**, eine Steuereinheit **14**, einen Drucksensor **30** und einen Akkumulator **16**.

Die zweite Kammer **4** enthält ein inhalierbares Hilfsmaterial, welches als Gas oder in flüssiger Form vorliegt. Vorzugsweise ist das Hilfsmaterial ein Liquid für eine E-Zigarette. Das Liquid kann eine Mischung sein, umfassend aromatische Elemente und Wirkstoffe. Der Verdampfer **8** ist hinter der zweiten Kammer angeordnet und von ihr getrennt, beispielsweise durch eine Trennwand oder durch die Außenwände der zweiten Kammer. Die zweite Kammer kann als Kapsel vorgesehen sein. Der Verdampfer **8** umfasst ein Heizelement wie z. B. eine Spule, die so konfiguriert ist, dass sie das Hilfsmaterial verdampft. Der Verdampfer **8** kann optional mehrere Seiten der zweiten Kammer umwickeln.

Obwohl in der Figur nicht dargestellt, kann die Vorrichtung auch ein Kühlsystem enthalten, wie z. B. eine Luftspule, die um das Mundstück oder zwischen der zweiten Kammer und dem Mundstück angebracht ist. Das Kühlsystem ist so konfiguriert, dass der freigesetzte Dampf oder das Aerosol auf eine sichere Temperatur für die Inhalation gebracht wird.

Die Vorrichtung umfasst weiterhin eine erste Kammer **12** für Drucksauerstoff (d.h. unter Druck gelagerter Sauerstoff). Die Kammer ist mit einem ersten Ventil **10** verbunden, welches vorzugsweise ein Magnetventil ist. Das Ventil ist dazu eingerichtet, Sauerstoff mit einer einstellbaren Durchflussrate in Richtung des Mundstücks frei zu setzen. Die zweite Kammer **4** umfasst ebenfalls ein Ventil (nicht gezeigt), vorzugsweise ein Magnetventil. Vorzugsweise wird der Sauerstoff mit dem Hilfsmaterial in einem Mischventil gemischt, wo der Sauerstoff auch dazu beitragen kann, das Hilfsmaterial zu zerstäuben. Alternativ werden die zwei Fluiden (der Sauerstoff and das Hilfsmaterial) in dem Mundstück **2** vermischt.

Der Drucksensor **30** ist so konfiguriert, dass er erkennt, ob die Vorrichtung in Gebrauch ist. Der Sensor kann an einer beliebigen Stelle der Vorrichtung angebracht werden, z. B. neben der Steuereinheit **14** oder in einem Kanal, der den Auslass (Mundstück **2**) mit der zweiten Kammer **4** verbindet. Vorzugsweise befindet sich der Drucksensor im oder nahe dem Mundstück **2**. Der Sensor **30** kann insbesondere so konfiguriert sein, dass er einen Unterdruck erkennt, der anzeigt, dass der Benutzer über das Mundstück inhaliert.

Die Steuereinheit **14** ist so konfiguriert, dass sie eines oder beide Ventile öffnet, um Flüssigkeit aus der ersten und/oder zweiten Kammer freizugeben, wenn der Drucksensor **30** anzeigt, dass ein Benutzer inhaliert. Darüber hinaus ist die Steuereinheit **14** dazu eingerichtet, den Verdampfer **8** in Betrieb zu setzen, wenn einen Unterdruck ermittelt wird. Vorzugsweise wird der Öffnungsgrad der Ventile durch den Wert des Unterdrucks und/oder durch die gewünschte freizusetzende Endzusammensetzung bestimmt.

Die Vorrichtung kann auch einen weiteren Sensor **24** zur Erfassung der Sauerstoffkonzentration in der Umgebungsluft umfassen. Die Sauerstoffkonzentration kann der Steuereinheit **14** zugeführt werden, um die Durchflussrate des aus der ersten Kammer **12** abzugebenden Sauerstoffs zu bestimmen. Alternativ oder zusätzlich kann die Steuereinheit **14** binär entscheiden, ob das erste Ventil **10** während der Inhalation geöffnet werden soll oder nicht, je nachdem, ob der ermittelte Sauerstoffgehalt unter einen akzeptablen Schwellenwert gefallen ist.

Ein Akkumulator **16** speist die Steuereinheit **14**, Sensor **30** und Aktuator der Ventile mit Strom.

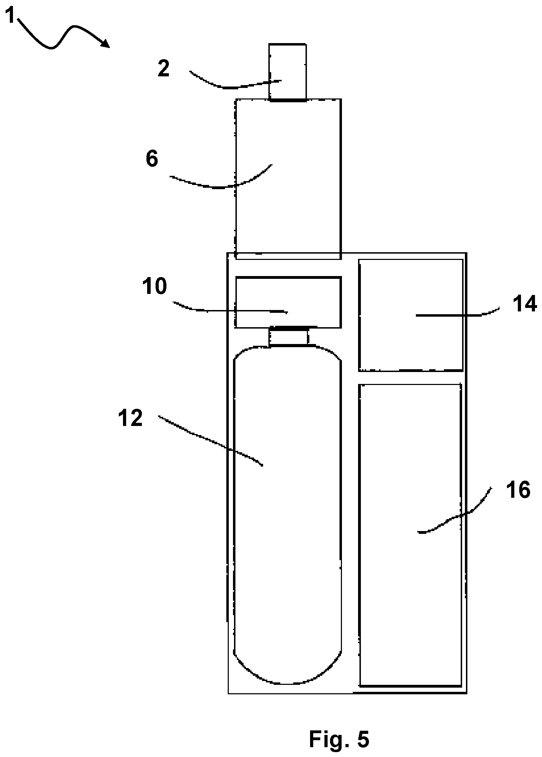

Die Figur 5 zeigt schematisch eine Vorrichtung **1** gemäß einer fünften bevorzugten Ausführungsform der Erfindung. Die Vorrichtung umfasst eine erste Kammer **12** und eine zweite Kammer **6.** Die zweite Kammer umfasst einen Verdampfer und ist dazu eingerichtet, ein Hilfsmaterial zu halten und zu verdampfen. Die zweite Kammer ist mit dem Mundstück **2** trennbar verbunden.

Die zweite Kammer **6** ist so gestaltet, dass sie lösbar mit einem Handgriff verbunden werden kann, vorzugsweise durch Schnappverschluss oder Verschraubung. Der Handgriff ist eine Einheit umfassend eine erste Kammer **12,** einen Akkumulator **16** und eine Steuereinheit **14.** Wenn die zweite Kammer **6** mit dem Handgriff verbunden ist, wird ein Flüssigkeitsverbindungskanal zwischen dem Mundstück und der ersten Kammer **12** hergestellt. Dieser kann standardmäßig durch das erste Ventil **10** geschlossen bleiben, bis die Öffnung des ersten Ventils **10** aktiviert wird.

Ähnlich wie bei der zweiten bevorzugten Ausführungsform umfasst die Vorrichtung vorzugsweise einen Drucksensor (nicht gezeigt), der so konfiguriert ist, dass er die Inhalation erkennt und einen Verdampfer **8** in der zweiten Kammer **6** aktiviert und das erste und/oder ein zweites Ventil öffnet, wenn der Benutzer inhaliert. Vorzugsweise sind die Komponenten des Handgriffs, einschließlich des Akkumulators **16** und der ersten Kammer **12**, so konfiguriert, dass sie vom Benutzer entfernt und ausgetauscht werden können. Sie können zum Beispiel mit einem Schnappverschluss in das Gehäuse des Handgriffs eingesetzt werden. Das Gehäuse des Handgriffs ist vorzugsweise so gestaltet und bemessen, dass es ergonomisch ist, wenn es von einem Benutzer gehalten wird.

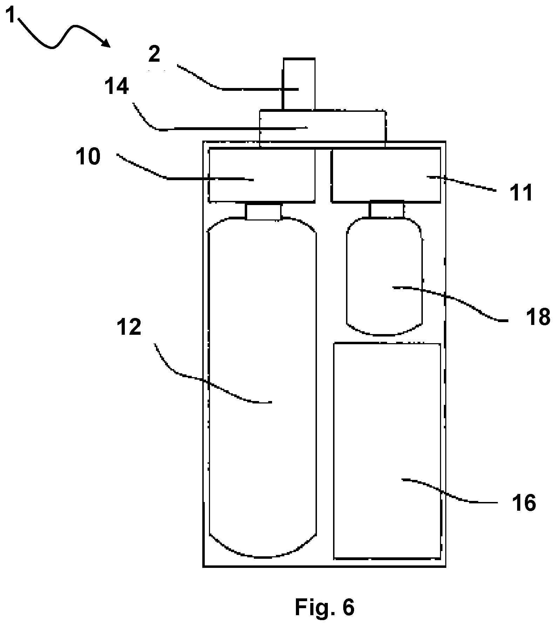

Die Figur 6 zeigt in schematischer Form eine Vorrichtung **1** gemäß einer sechsten bevorzugten Ausführungsform der Erfindung. Die Vorrichtung **1** umfasst eine erste Kammer **12** in Form einer Sauerstoffkapsel und eine zweite Kammer **18** in Form einer Aromakapsel. Die Aromakapsel enthält optional einen Wirkstoff. Die zwei Kapseln sind mittels Ventile **10, 11** mit einem Mundstück **2** verbunden. Das Mundstück **2** ist vorzugsweise abtrennbar von der Vorrichtung **1,** um gereinigt oder ersetzt zu werden. Die Steuereinheit **14** ist vorzugsweise zwischen dem Mundstück **2** und einem Handgriff angeordnet, wobei die Steuereinheit **14** Sensoren umfasst. Die Sensoren umfassen vorzugsweise mindestens einen Drucksensor und optional einen Sauerstoffsensor. Der Handgriff ist vorzugsweise als Einheit eingebildet und umhüllt die erste und zweite Kammer **12**, **18** sowie den Akkumulator **16.**

### BEZUGSZEICHENLISTE

- 1: - Vorrichtung
- 2: - Mundstück
- 4: - Liquid
- 6: - Liquid mit Verdampfer
- 8: - Verdampfer
- 10: - Erstes Ventil
- 11: - Zweites Ventil
- 12: - Sauerstoffkapsel
- 14: - Steuereinheit mit Sensoren
- 16: - Akkumulator
- 18: - Kapsel mit Aroma/Duft oder Wirkstoffen
- 19: - Mischventil mit Düse
- 20: - Ein-/Ausschalter (optional mit Zeitschaltuhr)
- 22: - Düse
- 24: - Sauerstoffsensor für Raumluft
- 26: - Stecker für Wechselstrom
- 28: - Mischkanal
- 29: - Transformer
- 30: - Drucksensor
- 31: - Elektroanschluss

## Patentansprüche

1. Vorrichtung (1) für die Anreicherung von Luft mit Sauerstoff umfassend eine Steuereinheit (14) und mindestens einen Sensor (24, 30)
**dadurch gekennzeichnet, dass**
die Vorrichtung (1) eine erste Kammer (12) und eine zweite Kammer (4, 6, 18) umfasst,
- wobei die erste Kammer (12) dafür konfiguriert ist, Sauerstoff unter Druck zu halten und als Reaktion auf ein erstes Signal der Steuereinheit (14) den Sauerstoff mit einem ersten Volumenstrom abzugeben,
- die zweite Kammer (4, 6, 18) dafür konfiguriert ist, als Reaktion auf ein zweites Signal der Steuereinheit einen inhalierbaren Dampf oder Aerosol mit einem zweiten Volumenstrom abzugeben, und
- der erste Volumenstrom größer als der zweite Volumenstrom ist.

2. Vorrichtung (1) gemäß Anspruch 1
**dadurch gekennzeichnet, dass**
die zweite Kammer (4, 6, 18) dafür konfiguriert ist, einen Wirkstoff und/oder ein Duft zu lagern, wobei der Wirkstoff und/oder der Duft vorzugsweise in flüssiger Form gelagert sind.

3. Vorrichtung (1) gemäß einem der vorherigen Ansprüche
**dadurch gekennzeichnet, dass**
die Vorrichtung (1) einen Auslass (2, 22) umfasst, wobei der Auslass mit der ersten Kammer (12) in Fluidverbindung steht, vorzugsweise mittels eines ersten Ventils (10), und der Auslass mit der zweiten Kammer (4) in Fluidverbindung steht, vorzugsweise mittels eines zweiten Ventils (11).

4. Vorrichtung gemäß Anspruch 3
**dadurch gekennzeichnet, dass**
das erste Ventil (10) und/oder das zweite Ventil (11) Magnetventile sind.

5. Vorrichtung gemäß einem der vorherigen Ansprüche
**dadurch gekennzeichnet, dass**
die Steuereinheit (14) so konfiguriert ist, dass sie das erste (10) und/oder das zweite Ventil (11) in Reaktion auf Signale von dem mindestens einen Sensor öffnet und/oder schließt.

6. Vorrichtung (1) gemäß dem vorherigen Anspruch
**dadurch gekennzeichnet, dass**
die zweite Kammer (4) einen Verdampfer (8) für die Verdampfung der zweiten Flüssigkeit umfasst.

7. Vorrichtung (1) gemäß einem der vorherigen Ansprüche
**dadurch gekennzeichnet, dass**
die Vorrichtung (1) einen Verdampfer (8) für die Verdampfung der zweiten Flüssigkeit umfasst, wobei der Verdampfer (8) durch eine Trennwand von der zweiten Kammer (4) getrennt vorliegt.

8. Vorrichtung (1) gemäß einem der vorherigen Ansprüche
**dadurch gekennzeichnet, dass**
der mindestens eine Sensor ein Drucksensor ist, der so konfiguriert ist, dass er das Ansaugen aus dem Auslass (2, 22) detektiert, und die Steuereinheit (14) so konfiguriert ist, dass sie das erste und das zweite Ventil (11) öffnet, wenn ein Unterdruck detektiert wird.

9. Vorrichtung (1) gemäß einem der vorherigen Ansprüche
**dadurch gekennzeichnet, dass**
der Auslass als Mundstück (2) oder als Zerstäuber (22) konfiguriert ist.

10. Vorrichtung (1) gemäß einem der vorherigen Ansprüche
**dadurch gekennzeichnet, dass**
die Vorrichtung einen Akkumulator (16) umfasst.

11. Vorrichtung (1) gemäß einem der vorherigen Ansprüche
**dadurch gekennzeichnet, dass**
die Vorrichtung Mittel (26, 31) zum Anschluss der Vorrichtung an das Stromnetz umfasst.

12. Vorrichtung (1) gemäß einem der vorherigen Ansprüche
**dadurch gekennzeichnet, dass**
die Vorrichtung weiterhin einen Sauerstoffsensor (24) umfasst, wobei die Steuereinheit (14) dafür konfiguriert ist, das erste Ventil (10) zu öffnen wenn ein ermittelter Sauerstoffgehalt unter ein vorgegebenes Niveau fällt.

13. Vorrichtung (1) gemäß einem der vorherigen Ansprüche
**dadurch gekennzeichnet, dass**
die Vorrichtung (1) ferner ein Mischventil (19) umfasst, der sowohl mit der ersten (10) und der zweiten Kammer (11) als auch mit dem Auslass (2, 22) in Fluidverbindung steht.

14. Verwendung einer Vorrichtung (1) gemäß einem der vorherigen Ansprüche zur Verbesserung der Luftqualität in einem Raum.

15. Bausatz für die Anreicherung von Luft mit Sauerstoff, wobei der Bausatz Folgendes umfasst:
- einen Akkumulator (16) und/oder einen Stromnetzanschluss (26, 31),
- eine erste Kammer (12) mit komprimiertem Sauerstoff,
- eine zweite Kammer (18) mit einem Hilfsstoff,
- eine Steuereinheit (14),
- mindestens einen Sensor (24, 30) und
- ein Mundstück (2) oder einen Zerstäuber (22).
